# EUROPÄISCHE PATENTSCHRIFT

(11) **EP 4 149 389 B1**
(45) Veröffentlichungstag und Bekanntmachung des Hinweises auf die Patenterteilung: **15.05.2024**
(21) Anmeldenummer: 21725151.1
(22) Anmeldetag: 11.05.2021
(51) Int. Cl.: A61F 2/08, A61B 17/064

(54) **SEHNEN-FIXATIONSPLATTE**
TENDON FIXATION PLATE
PLAQUE DE FIXATION DE TENDON

(30) Priorität: 14.05.2020 DE 102020113146
(43) Veröffentlichungstag der Anmeldung: 22.03.2023
(73) Patentinhaber: INOVEDIS GmbH, 72461 Albstadt (DE)
(72) Erfinder: FLÖSS, Lukas, 72513 Inneringen (DE); WELTE, Stefan, 72458 Albstadt (DE)
(74) Vertreter: Otten, Roth, Dobler & Partner mbB Patentanwälte
(86) Internationale Anmeldenummer: PCT/EP2021/062411
(87) Internationale Veröffentlichungsnummer: WO 2021/228807

(56) Entgegenhaltungen:
- EP-A1- 0 358 372
- WO-A1-2019/157853
- US-A1- 2007 118 128
- US-A1- 2013 096 678
- US-A1- 2016 100 932
- US-A1- 2016 100 933
- US-A1- 2016 242 771
- US-A1- 2017 181 840

## Beschreibung

Die Erfindung betrifft ein Implantat zur flächigen Verbindung von Gewebe mit Knochen nach dem Oberbegriff des Anspruchs 1.

Ein medizinischer Eingriff zur Fixierung und Rekonstruktion von abgetrenntem Gewebe, wie beispielsweise Sehnengewebe, ist ein gängiges Verfahren und kann verschiedene Ursachen haben. Aus dem Stand der Technik ist diesbezüglich bekannt, dass zur Rekonstruktion, beispielsweise einer abgetrennten Sehne, das abgetrennte Sehnengewebe mittels eines Implantats zur Einheilung an einem Knochen an diesem Knochen fixiert wird. Hierbei muss unter Anderem eine suffiziente Durchblutung des Sehnengewebes zur Verhinderung von nekrotischen Prozessen gewährleistet sein.

Ein solches Implantat ist beispielsweise aus der EP 3 184 078 B1 bekannt. Hier wird das abgetrennte Sehnengewebe mittels einer durchbrochenen Klemmfläche und wenigstens drei daran verbundenen Befestigungsmittel dauerhaft an dem Knochen fixiert. Das Implantat wird zur Fixierung mittels eines Spezialwerkzeugs gehalten und die Befestigungsmittel werden in den Knochen z.B. mittels eines Schlagwerkzeugs eingetrieben. Das Gewebe wird dadurch flächig mit dem Knochen verbunden und angepresst.

Nachteilig ist hierbei insbesondere, dass, für einen sicheren Halt, das Implantat mit seinen Befestigungsmitteln äußerst präzise in den Knochen eingetrieben werden muss. Position, Winkel und Orientierung, in denen das Implantat befestigt wird, müssen sorgfältig gewählt werden, was zu einer Erhöhung des Zeitaufwands und den damit einhergehenden den Kosten bei einem solchen chirurgischen Eingriff sorgt.

Ein fehlerhaftes Eintreiben in den Knochen kann sich überdies auf die Stabilität des Implantats auswirken und ein Lösen des Gewebes vom Knochen begünstigen. Dadurch wird ein erneuter Eingriff notwendig, was eine zusätzliche Belastung des Patienten darstellt.

Überdies ist oftmals eine minimalinvasive Implantation im chirurgischen Verfahren, aufgrund der erheblichen Größe des Implantats, nicht möglich.

Weiterhin ist aus der WO 2019/157853 A1 eine Fixationsklammer für die Kniegelenkchirurgie zum Fixieren des Seitenbandes des Kniegelenks oder zum Verhindern der Verletzung der Patellasehne am Tuberculum tibialis bekannt.

Weiterhin ist aus der EP 0 358 372 A1 eine Kanal-Bandklemme, umfassend einen Abschnitt eines Kanals der aus einem Material hergestellt ist, das für eine menschliche Implantation geeignet ist, mit aufrechten, beabstandeten parallelen Dornen, bekannt.

Weiterhin sind aus der US 2007/118128 A1 verschiedene exemplarische Verfahren und Vorrichtungen zum Fixieren eines Implantats an nativem Gewebe, wie beispielsweise Knochen, bekannt.

Weiterhin sind aus der US 2013/096678 A1 Transplantatfixierungsanordnungen bekannt, die ein Basiselement, ein Kompressionslement und ein Befestigungselement umfassen.

Weiterhin ist aus der US 2016/242771 A1 eine Knochenfixationsvorrichtung oder Knochenklammer bekannt, umfassend einen Kronenabschnitt, einen Eingriffsabschnitt und einen äußeren Rand, der allgemein zwischen dem Kronenabschnitt und dem Eingriffsabschnitt definiert ist.

Aufgabe der Erfindung ist es daher, eine Befestigungsmöglichkeit von Gewebe mit Knochen im Rahmen eines operativen Eingriffs bereitzustellen, welche unter Minimierung von nekrotischen Prozessen stabil und einfacher unter vermindertem Zeit- und Kostenaufwand durchführbar ist.

Diese Aufgabe wird in Verbindung mit dem Oberbegriff des Patentanspruchs 1 erfindungsgemäß durch die kennzeichnenden Merkmale des Patentanspruchs 1 gelöst. In den Unteransprüchen sind vorteilhafte und zweckmäßige Weiterbildungen angegeben.

Auf diese Weise kann eine möglichst große Auflagefläche bzw. Klemmfläche und Symmetrie des Implantats zu seiner Befestigung bereitgestellt werden, ohne dass ungleichmäßige Verhältnisse im Anpressdruck, z.B. in ausgeprägten Eckbereichen, auftreten. Außerdem bietet diese Formgebung des Implantats Einsatzmöglichkeiten im Bereich der minimalinvasiven Chirurgie, da es mittels minimalinvasiv agierender Werkzeuge gehandhabt und durch entsprechende Geräte hindurch eingebracht und positioniert werden kann.

Das Befestigungsmittel kann im Zusammenwirken mit der Klemmfläche und dem Knochen optimal angepasst werden. Außerdem ist es möglich das Eintreiben des Befestigungsmittels in den Knochen mittels eines Spezialwerkzeugs, beispielsweise eines Schraubendrehers oder eines Schlagadapters zu ermöglichen.

Zudem kann durch das Implantat ein kontinuierlicher Anpressdruck erreicht werden, der über den gesamten Zeitraum einer Einheilung aufrechterhalten werden kann. Insofern kann eine zügigere Einheilung ermöglicht werden.

Der benötigte Zeitaufwand kann dadurch bei einem operativen Eingriff signifikant reduziert und der Heilungsprozess verkürzt werden. In vorteilhafter Weise kann somit eine Behandlungsdauer nach einem operativen Eingriff, sowie Behandlungskosten reduziert werden, da Nachbehandlungen deutlich erleichtert werden können. Etwaige Behandlungen nach einem Einbringen des Implantats bzw. einer Verbindung von Gewebe und Knochen können dadurch verringert werden oder gar gänzlich ausbleiben.

Weiterhin ist vorgesehen, dass die weitere Öffnung in der Klemmfläche zentriert ist.

Hierdurch kann eine optimale Ausrichtung und Platzierung des Implantats an gewünschter Stelle ermöglicht werden, was den dazugehörigen Eingriff erleichtert. Das Implantat kann somit beispielsweise in verschiedenen Orientierungen am Knochen befestigt werden, was einen geringeren Zeitaufwand und eine höhere Flexibilität für den Operateur bedeutet, da während des Eingriffs dadurch schneller eine optimale Ausrichtung gefunden werden kann.

Außerdem ist vorgesehen, dass die weitere Öffnung eine Senke zur formschlüssigen Aufnahme des Kopfes aufweist.

Hierdurch wird eine gleichmäßige und schnellere Befestigung der Klemmfläche am Knochen ermöglicht. Der Kopf des Befestigungsmittels kann hierdurch formschlüssig mit dem Implantat verbunden werden und ein definierter Halt, sowie eine definierte Ausrichtung zwischen Befestigungsmittel und Implantat ermöglicht werden, ohne dass hierfür weitere Maßnahmen notwendig wären.

Weiterhin ist vorgesehen, dass der Hals und der Kopf mit der Öffnung ein Gelenk, insbesondere ein taumelgelenkartiges Gelenk, ausbilden.

Hierdurch wird ermöglicht, dass die Klemmfläche gegenüber dem Befestigungsmittel verschiedene Lagewinkel einnehmen kann. Die Klemmfläche ist somit flexibel mit dem Befestigungsmittel verbunden und ein Anliegen an den Knochen wird erleichtert.

Dies erfolgt durch ein Verkippen der Klemmfläche gegenüber dem Knochen, was eine größere Flexibilität bei der Richtung des Einbringens des Implantats und beim Fixieren des Gewebes ermöglicht.

Unebenheiten und anatomische Unterschiede bei dem Knochen können dadurch überdies ausgeglichen und ein optimales Anliegen der Klemmfläche ermöglicht werden. Dadurch ist eine größere Flexibilität bei der Wahl einer Befestigungsstelle für das Implantat gegeben und sie kann bestmöglich ausgewählt werden.

Außerdem ist vorgesehen, dass das Gelenk ein Verkippen der Klemmfläche zu einer durch die Längsachse des Stiftes definierten Achse um zwischen 0° und 65°, insbesondere 0° und 55°, bevorzugt 0° und 45°, besonders bevorzugt zwischen 0° und 20° ermöglicht.

Dadurch wird das Verkippen der Klemmfläche zur Längsachse des Stiftes eingeschränkt und ein Winkelbereich geschaffen, in welchem die Klemmfläche der Sehne einerseits optimalen Halt geben und andererseits die nötige Flexibilität beim Einbringen und Befestigen aufweist.

Außerdem ist vorgesehen, dass die weitere Öffnung in der Klemmfläche exzentrisch ist.

Hierdurch bietet sich für einen Operateur die Möglichkeit, die Lage der Klemmfläche relativ zu dem Befestigungsmittel und der zu fixierenden Sehne zu wählen, was eine Erhöhung der Flexibilität bei der Suche nach einer geeigneten Implantationsstelle ermöglichen kann.

Weiterhin ist vorgesehen, dass die Klemmfläche wenigstens einen Ausleger umfasst, wobei der Ausleger zu der Klemmfläche gekröpft ausgebildet ist, wobei der Ausleger wenigstens eine Öffnung aufweist, wobei die Öffnung des Auslegers zur Aufnahme eines Befestigungsmittels ausgebildet ist und wobei eine weitere Öffnung des Auslegers als Aussparung ausgebildet ist.

Dadurch kann eine weitere Befestigungsmöglichkeit des Implantates bereitgestellt werden. Das Einbringen des Implantates bei einem operativen Eingriff kann zudem vereinfacht werden. Beispielsweise kann ein weiteres Befestigungsmittel herangezogen werden, welches in der Öffnung des Auslegers angeordnet ist und Gewebe und Knochen miteinander verbinden. Es kann auch ermöglicht werden dass das Implantat mit Gewebe und zusätzlich mit dem Knochen befestigbar ist. Weiterhin kann die Aussparung eine Durchblutung und eine Sauerstoffversorgung der Sehne begünstigen und somit den Heilungsprozess begünstigen.

Weiterhin ist vorgesehen, dass die Klemmfläche wenigstens zwei sich gegenüberliegende Dorne umfasst, wobei die Dorne an der Unterseite der Klemmfläche ausgebildet sind und/oder wobei die Dorne bevorzugt Widerhaken umfassen.

Die Dorne können eine zusätzliche Befestigungsmöglichkeit beispielsweise für eine Vorabbefestigung des Implantats bieten. Außerdem können sie in einer Art ausgebildet sein, um ein zu tiefes Eintreiben des Befestigungsmittels und einen dadurch zu hohen Anpressdruck der Klemmfläche zu verhindern. Die Dornen ermöglichen dadurch ein schnelleres und sichereres Einbringen des Implantats.

Weiterhin ist vorgesehen, dass der Stift ein Gewinde umfasst.

Hierdurch kann ein sicherer und bei Bedarf wieder lösbarer Halt des Befestigungsmittels ermöglicht werden.

Außerdem ist vorgesehen, dass der Stift hülsenförmig, insbesondere nach der Art einer Einschlagshülse ausgebildet ist und/oder dass der Stift Widerhaken umfasst.

Hierdurch kann das Einbringen des Befestigungsmittels in den Knochen mittels eines geeigneten Werkzeugs, beispielsweise eines gehärteten Schlagwerkzeugs ermöglicht werden.

Weiterhin ist vorgesehen, dass die Klemmfläche mehrere Fixiermittel umfasst, wobei die Fixiermittel an einer Unterseite der Klemmfläche, insbesondere senkrecht zu der Klemmfläche ausgebildet, sind.

Die Fixiermittel geben der Sehne zusätzlichen Halt an dem Knochen. Die Sehne wird zwischen Klemmfläche und Knochen gehalten. Dadurch dass die oben genannte kreisrunde oder ovale oder vieleckige, insbesondere rotationssymmetrische Formgebung mehr Möglichkeiten zur Orientierung des Implantats liefert, müssen die Fixiermittel stets bestmöglich in der Sehne angreifen können.

Die senkrechte Ausbildung der Fixiermittel ermöglicht die Gewährleistung der Flexibilität beim Einsetzen des Implantats und die Stabilität beim Fixieren der Sehne. Die Fixiermittel verhindern ein Durchrutschen des Gewebes, beispielsweise einer Sehne, und sorgen für einen sicheren bzw. stabilen Halt der Sehne bei geringem Anpressdruck der Klemmfläche. Ein schnelleres Einheilen kann somit ermöglicht werden, wobei nekrotische Prozesse vermieden werden können.

Außerdem ist vorgesehen, dass die Fixiermittel material-einstückig an der Klemmfläche ausgebildet sind und/oder dass die Fixiermittel kegelförmig ausgebildet sind und/oder dass die Fixiermittel flächendicht ausgebildet sind.

Hierdurch wird eine stabile Verbindung zwischen Fixiermittel und Klemmfläche gewährleistet, was den sicheren Halt der Sehne an dem Knochen unterstützt. Außerdem ist dadurch eine einfache Herstellung des Implantats ermöglicht, was den Kostenaufwand der Herstellung und somit des chirurgischen Eingriffs verbessert.

Es kann vorgesehen sein, dass zumindest die Klemmfläche und die Fixiermittel aus einem Kunststoff, insbesondere einem biokompatiblen Kunststoff gefertigt sind. Einerseits weisen solche Materialien eine ausreichende Stabilität auf, um Gewebe und Knochen miteinander zu verbinden. Andererseits sind derartige Materialien für den menschlichen Körper gut verträglich.

Die Kegelform lässt ein schnelles Eindringen der Fixiermittel in die Sehne zu. Hierdurch kann die Sehne leichter durch das Implantat gehalten werden und das Implantat bei dem operativen Eingriff beschleunigt eingebracht werden. Dadurch kann der Zeitaufwand und der Kostenaufwand verringert werden.

Dadurch dass die Fixiermittel flächendicht ausgebildet sind, wird die Unterseite der Klemmfläche gleichmäßig mit zueinander beabstandeten Fixiermitteln ausgestattet, um so eine möglichst gute Flächenhaftung an der Sehne zu gewährleisten. Dies sorgt für eine Vergrößerung der Angriffsfläche des Implantats gegenüber des zu fixierenden Gewebes, beispielsweise einer Sehne. Eine hohe Anzahl auf der gesamten Unterseite der Klemmfläche erhöht die Fixierwirkung des Implantats und ermöglicht eine Verringerung des Anpressdrucks. Außerdem wird dadurch ein Durchrutschen des Gewebes, beispielsweise einer Sehne, verhindert.

Weiterhin ist vorgesehen, dass die Fixiermittel mit einer Länge von mehr als 2 Millimeter, bevorzugt mit einer Länge zwischen 2 und 6 Millimeter ausgebildet sind und/oder dass die Länge der Fixiermittel zum äußeren Rand der Klemmfläche zunimmt.

Diese Längen sind besonders vorteilhaft für die Fixierung von Gewebe, beispielsweise von Sehnen, insbesondere von Sehnen im Bereich der menschlichen Schulter. Eine bestimmte Länge der Fixiermittel verhindert durch ihre Durchdringung des Gewebes und der danach folgenden Abstützung am Knochen einen zu hohen Anpressdruck des Implantats und gewährleistet gleichzeitig eine besonders sichere bzw. stabile Fixierung von Gewebe auf Knochen.

Durch die längeren Fixiermittel am äußeren Rand der Klemmfläche können Unterschiede im Anpressdruck auf Grund der Zentrierung des Befestigungsmittels ausgeglichen werden. Dies sorgt für verbesserte Eigenschaften beim Fixieren der Sehne.

Es ist dadurch auch möglich, individuelle anatomische Gegebenheiten und Unebenheiten des Knochens zu berücksichtigen. Dadurch kann das Einbringen des Implantats bzw. das Verbinden von Gewebe und Knochen vereinfacht bzw. beschleunigt werden. Zudem kann eine erhöhte Stabilität des Halts von Gewebe und Knochen erreicht werden. Es kann dadurch ferner ein gleichmäßigeres Anpressen des Gewebes auf der gesamten Fläche der Klemmfläche erreicht werden.

Außerdem ist vorgesehen, dass wenigstens zwei Befestigungsmittel in etwa parallel zueinander angeordnet sind und/oder unterschiedliche Längen aufweisen und/oder dass die Klemmfläche eine Ebene bildet, wobei wenigstens ein Befestigungsmittel in etwa senkrecht zu der Ebene und/oder wenigstens ein Befestigungsmittel in etwa parallel zu der Ebene angeordnet ist.

Durch die unterschiedliche Ausbildung bzw. Anordnung der Befestigungsmittel können individuelle anatomische Gegebenheiten berücksichtigt werden. Daraus kann ein reduzierter Zeitaufwand bei einem operativen Eingriff resultieren. Daraus kann weiterhin ein verbesserter Halt des Implantates und damit einhergehend eine feste Verbindung zwischen Gewebe und Knochen resultieren.

Weiterhin ist vorgesehen, dass die Klemmfläche eine Werkzeugangriffsfläche und/oder Werkzeugangriffsöffnung umfasst, wobei die Angriffsfläche frei von Fixiermitteln ist.

Ein stabiles Greifen des Implantats zur Befestigung am Knochen kann einen zeitsparenden und sicheren chirurgischen Eingriff ermöglichen. Die Lage und Orientierung kann durch das bevorzugt formschlüssige Angreifen beispielsweise mittels eines Spezialwerkzeugs, z.B. einer Zangenhalterung mit innenliegender Werkzeugdurchführung insbesondere aus dem Bereich der minimalinvasiven Chirurgie präzise gewählt werden.

Das Fehlen von Fixiermitteln an dieser Angriffsfläche ist dabei von Vorteil, da das Werkzeug dadurch einfacher ausgestaltet und das Implantat sicherer gehalten werden kann. Das Einbringen des Implantats bei einem operativen Eingriff kann dadurch vereinfacht und beschleunigt werden.

Außerdem ist vorgesehen, dass der wenigstens eine Ausleger etwa entlang einer Quererstreckung der Klemmfläche angeordnet ist und/oder dass der wenigstens eine Ausleger und die Klemmfläche einstückig ausgestaltet sind.

Dies stellt eine weitere Befestigungsmöglichkeit des Implantates, beispielsweise am Knochen, dar. Dadurch kann ermöglicht werden, dass die Verbindung zwischen Gewebe und Knochen nach einem Einbringen des Implantates erhöhten Belastungen standhält. Somit können Behandlungen nach einem Verbinden von Gewebe und Knochen mittels des Implantates ausbleiben.

Weiterhin ist vorgesehen, dass die Klemmfläche einen zumindest abschnittsweise gerundeten Umfang aufweist und/oder zumindest abschnittsweise gewölbt ist.

Es ist somit möglich, individuelle anatomische Gegebenheiten zu berücksichtigen. Dadurch kann das Einbringen des Implantates bzw. das Verbinden von Gewebe und Knochen vereinfacht bzw. beschleunigt werden. Zudem kann ein stabiler Halt von Gewebe und Knochen erreicht werden. Es wird dadurch ferner ein gleichmäßigeres Anpressen des Gewebes auf der gesamten Fläche der Klemmfläche erreicht. Vorteilhaft ist bei einer gewölbten Form der Klemmfläche dass ein einfaches Modellieren des Implantates an den Knochen ermöglicht werden kann.

Im Sinne der Erfindung können die Fixiermittel auch als so genannte Spikes oder Zähnchen bezeichnet werden.

Weiterhin kann im Sinne der Erfindung das Befestigungsmittel auch als Schraube, Nagel, oder Knochenanker bezeichnet und aufgefasst werden. Derartige Befestigungsmittel sind kostengünstig produzierbar und ermöglichen eine stabile Verbindung zwischen Knochen und Implantat.

Außerdem kann im Sinne der Erfindung unter einer Einschlagshülse eine Hülse mit konusförmiger Spitze verstanden werden, die das Eindringen der Hülse in ein festes Objekt, beispielsweise einen Knochen vereinfacht.

Weitere Einzelheiten der Erfindung werden in den Zeichnungen anhand von schematisch dargestellten Ausführungsbeispielen beschrieben.

Hierbei zeigen
- Figur 1: eine perspektivische Darstellung eines Implantats in einer nicht zur Erfindung gehörenden Ausführungsform mit einer Schraube als Befestigungsmittel;
- Figur 2: eine Draufsicht eines Implantats in einer nicht zur Erfindung gehörenden Ausführungsform mit sichtbarem Kopf einer Schraube und
- Figur 3: eine perspektivische Darstellung eines erfindungsgemäßen Implantats mit Ausleger und zwei Befestigungsmitteln.

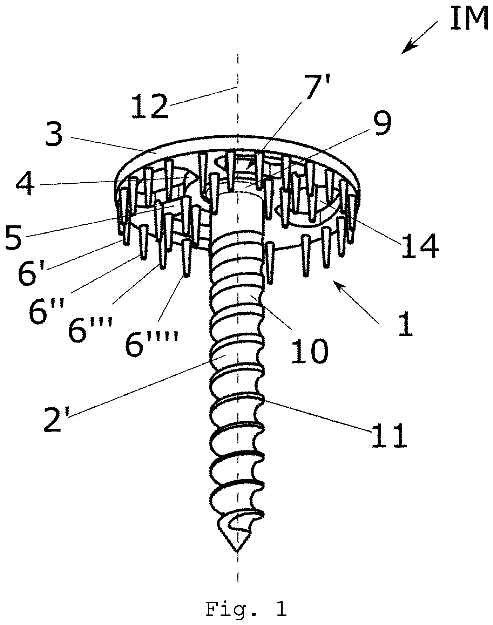

In der Figur 1 ist eine perspektivische Darstellung eines Implantats IM in einer nicht zur Erfindung gehörenden Ausführungsform zur flächigen Verbindung von Gewebe mit Knochen gezeigt. Dies umfasst eine Klemmfläche 1 unter der das Gewebe, beispielsweise Sehnengewebe, fixiert und zum Einheilen an den Knochen gehalten wird. Die Klemmfläche 1 umfasst einen äußeren Rand 3 und einen inneren Rand 4, wobei der äußere Rand 3 mittels Verbindungsstegen 5 mit dem inneren Rand 4 verbunden ist. Die Klemmfläche 1 umfasst weiterhin in ihr angeordnete Öffnungen 14, die mittels der Verbindungsstege 5 und dem äußeren Rand 3 der Klemmfläche 1 umschlossen sind. Die Öffnungen 14 ermöglichen eine für den Heilungsprozess suffiziente Durchblutung des Sehnengewebes. Gleichzeitig kann dadurch nekrotischen Prozessen vorgebeugt werden, da die Klemmfläche 1 auf der Sehne flächig aufliegt und Spitzen des Anpressdrucks vermieden werden.

Die Klemmfläche 1 bildet eine Ebene und ist in dem in Figur 1 gezeigten Ausführungsbeispiel mit einem kreisrunden Umfang ausgebildet. Insbesondere kann die Klemmfläche 1 auch mit einer ovalen, vieleckigen, rotationssymmetrischen und/oder spiegelsymmetrischen Kontur ausgebildet sein. Durch derartige Formen ist ein großflächiges Anliegen der Klemmfläche 1 an der Sehne ermöglicht und der Anpressdruck wird gleichmäßig verteilt.

Die Klemmfläche 1 weist eine zentrierte Öffnung 7' auf. Diese ist zur Aufnahme eines Befestigungsmittels 2' ausgebildet. Mittels des Befestigungsmittels 2' kann das Implantat IM stabil und sicher am Knochen befestigt werden.

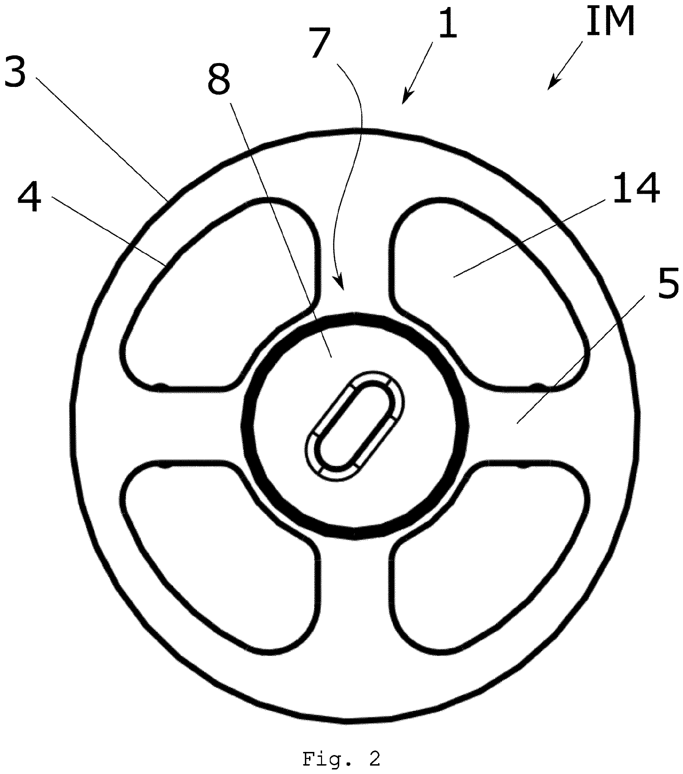

In diesem Ausführungsbeispiel ist das Befestigungsmittel 2' als Schraube 11, umfassend einen gewindeförmigen Stift 10, einen Kopf 8 (in Figur 2 dargestellt) und einen Hals 9, ausgebildet. Eine Schraube 11 bietet den Vorteil durch Drehbewegung die Eindringtiefe in den Knochen bestimmen zu können. Dadurch bietet sich die Möglichkeit den Anpressdruck der Klemmfläche auf das zu fixierende Gewebe, beispielsweise einer Sehne, optimal einzustellen. Einerseits kann darüber hinaus ein zu tiefes Befestigen des Implantats IM, was ggf. zu einem überhöhten Anpressdruck führt, durch Zurückdrehen korrigiert werden, andererseits ist durch eine Schraube 11 ein nachträgliches Lösen des Implantats IM ermöglicht. Eine Schraube 11 kann darüber hinaus mittels eines Dübels in einem Knochen verankert werden, wodurch ein verbesserter Halt im Knochen erreicht werden kann.

Der Hals 9 und der Kopf 8 (in Figur 2 dargestellt) bilden mit der zentrierten Öffnung 7' ein taumelgelenkartiges Gelenk, wodurch sich die Lage der Klemmfläche 1 zur Längsachse des Stifts 12 bei der Befestigung des Implantats IM kippen lässt. Dadurch wird ein optimales Anliegen und Fixieren des Gewebes ermöglicht und das Implantat IM kann sich auf anatomische Gegebenheiten flexibel anpassen.

Dargestellt sind außerdem Fixiermittel, exemplarisch 6', 6", 6‴, 6ʺʺ, die das Fixieren von Gewebe, beispielsweise Sehnengewebe, ermöglichen und ein Verbinden bzw. Befestigen von Gewebe, beispielsweise Sehnengewebe, an Knochen vereinfachen bzw. die Verbindung von Gewebe und Knochen verstärken. Die Fixiermittel 6', 6", 6‴, 6ʺʺ sind an der Unterseite der Klemmfläche 1 ausgebildet. Dadurch kann das Gewebe zwischen der Klemmfläche 1 und dem Knochen gehalten werden.

Die Fixiermittel 6', 6", 6‴, 6ʺʺ sind senkrecht zu der Klemmfläche 1 ausgebildet, wodurch das Implantat IM dem Gewebe, z.B. einer Sehne, einen stabilen und festen Halt geben kann. Die Fixiermittel 1 sind kegelförmig ausgebildet, wodurch sie einfach in das zu fixierende Gewebe eindringen und somit ein vereinfachtes Anbringen des Implantats IM ermöglicht wird.

Die Fixiermittel 6', 6", 6‴, 6ʺʺ sind material-einstückig an der Klemmfläche 1 ausgebildet, sodass eine stabile Verbindung zwischen Fixiermittel 6', 6", 6‴, 6ʺʺ und Klemmfläche 1 besteht und das Implantat IM einfach und schnell hergestellt werden kann.

Indem die Fixiermittel 6', 6", 6‴, 6ʺʺ flächendicht an der Unterseite der Klemmfläche 1 ausgebildet sind, können sie dem Gewebe einen verbesserten gleichmäßigen Halt ausbilden. Die Länge der Fixiermittel 6', 6", 6‴, 6ʺʺ ist von mehr als 2 Millimeter, bevorzugt mit einer Länge von 2 bis 6 Millimeter ausgebildet, damit das Gewebe, beispielsweise eine menschliche Schultersehne, optimal gehalten werden kann.

Figur 2 zeigt eine Draufsicht der in Figur 1 gezeigten und nicht zur Erfindung gehörenden Ausführungsvariante des Implantats IM mit einem äußeren Rand 3 und einem inneren Rand 4. Der kreisrunde Umfang der Klemmfläche 1 ermöglicht die Anwendung bei minimalinvasiven chirurgischen Eingriffen. Der Kopf 8 des Befestigungsmittels verdeckt in dieser Draufsicht der Figur 2 die Öffnung 7 (nicht sichtbar) des Implantats IM.

Das Befestigungsmittel 2', das hier als Schraube 11 ausgebildet ist, kann mittels eines geeigneten Spezialwerkzeugs in einen Knochen eingedreht werden. Ein schnelles und einfaches Einbringen des Implantats wird dadurch ermöglicht. Gleichzeitig kann durch das Eindrehen der Schraube 11 flexibel der Anpressdruck der Klemmfläche 1 auf das zu fixierende Gewebe verändert und eingestellt werden. Die Klemmfläche 1 ist von den auch in Figur 1 gezeigten Verbindungsstegen 5 und Öffnungen 14 durchbrochen, wodurch ein darunter fixiertes Gewebe, beispielsweise Sehnengewebe, suffizient durchblutet und nekrotische Prozesse gehemmt werden.

An den Verbindungsstegen 5 kann ein geeignetes Haltewerkzeug, oder Greifwerkzeug das Implantat IM bei einem chirurgischen Eingriff halten und optimal führen. Dadurch kann es an einer gezielten Stelle im Knochen eingebracht und befestigt werden.

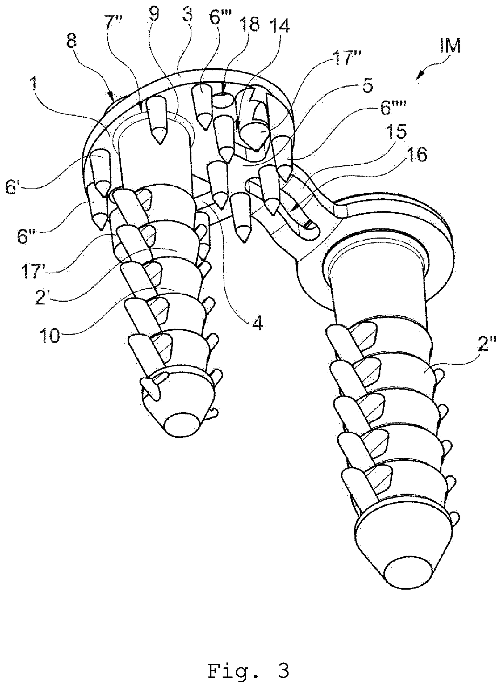

In Figur 3 ist eine Ausführungsvariante des Implantats IM mit Ausleger 15 in einer perspektivischen Darstellung von der Unterseite des Implantats gezeigt. Das Implantat IM zur flächigen Verbindung von Gewebe mit Knochen umfasst die Klemmfläche 1, wobei sie den äußeren Rand 3 und den inneren Rand 4 umfasst. Der äußere Rand 3 ist mittels eines Verbindungssteges 5 mit dem inneren Rand 4 verbunden und die Klemmfläche 1 umfasst innerhalb der Klemmfläche 1 angeordnete Öffnungen 14, 7", wobei die Öffnung 7" mittels eines Verbindungssteges 5 mit einem Teilbereich des äußeren Randes 3 verbunden ist.

Die Klemmfläche 1 weist eine ovale Kontur auf und die weitere Öffnung 7" ist exzentrisch in der Klemmfläche 1 zur Aufnahme eines Befestigungsmittels 2' ausgebildet. Hierdurch kann eine optimale Lage des Implantats IM relativ zu Sehne und Befestigungsmittel 2' gewählt werden. Das Befestigungsmittel 2' umfasst einen Stift 10, einen Kopf 8 (in dieser Darstellung nicht sichtbar), sowie einen Hals 9.

Die Klemmfläche 1 umfasst einen zu ihr abgekröpften Ausleger 15, wobei der Ausleger 15 Öffnungen 16, 19 aufweist, wobei die Öffnung 19 des Auslegers zur Aufnahme eines Befestigungsmittels 2" ausgebildet ist und wobei eine weitere Öffnung 16 des Auslegers als Aussparung ausgebildet ist. Der Ausleger 15 ist entlang einer Querstreckung der Klemmfläche 1 angeordnet und dieser einstückig ausgestaltet. Im Zusammenwirken mit der exzentrisch angeordneten Öffnung 7" der Klemmfläche 1 und des Befestigungsmittels 2' wird ein einfaches und schnelles Fixieren des Implantats IM am Knochen ermöglicht. Die als Aussparung ausgebildete weitere Öffnung 16 des Auslegers ermöglicht eine nur geringe Anlagefläche des Auslegers 15 an das teilweise von ihm bedeckte Gewebe und kann sich dadurch positiv auf die Verbesserung der Sauerstoffversorgung oder Durchblutung beitragen. Dadurch dass der Ausleger 15 gekröpft ausgebildet ist, können ein zu großer Anpressdruck der Klemmfläche 1 auf eine unter ihr zu haltende Sehne, sowie daraus resultierende nekrotische Effekte verhindert werden.

Ebenfalls ist in Figur 3 dargestellt, dass die Klemmfläche in die zwei an ihrer Unterseite ausgebildeten und sich gegenüberliegenden Dorne 17', 17" umfasst, wobei die Dorne 17', 17" ihrerseits Widerhaken umfassen. Der Stift 10 der Befestigungsmittel 2', 2"ist hülsenformig, nach der Art einer Einschlagshülse, ausgebildet und umfasst ebenfalls Widerhaken. Eine derartige Formgebung ermöglicht das einfache Einschlagen des Befestigungsmittels 2" in den Knochen und sichert dieses gegen ein ungewolltes Lösen.

Weiterhin ist in Figur 3 dargestellt, dass die Klemmfläche mehrere Fixiermittel 6', 6", 6‴, 6ʺʺ umfasst, wobei diese an der Unterseite der Klemmfläche 1, senkrecht zu ihr, mit ihr material-einstückig, sowie kegelförmig und flächendicht ausgebildet sind.

Darüber hinaus ist zu erkennen, dass das Befestigungsmittel der Klemmfläche 1, sowie das Befestigungsmittel 2" des Auslegers parallel zueinander angeordnet sind und unterschiedliche Längen aufweisen, sowie dass die Klemmfläche 1 eine Ebene bildet, wobei das Befestigungsmittel 2' der Klemmfläche senkrecht zu dieser Ebene angeordnet ist.

### Bezugszeichenliste:

- IM: Implantat
- 1: Klemmfläche
- 2': Befestigungsmittel (Klemmfläche)
- 2": Befestigungsmittel (Ausleger)
- 3: Rand (äußerer)
- 4: Rand (innerer)
- 5: Verbindungssteg
- 6` bis 6ʺʺ: Fixiermittel
- 7': Öffnung (zentrisch)
- 7": Öffnung (exzentrisch)
- 8: Kopf
- 9: Hals
- 10: Stift
- 11: Schraube
- 12: Längsachse des Stiftes
- 14: Öffnung
- 15: Ausleger
- 16: Öffnung (weitere, Ausleger)
- 17', 17": Dorn
- 18: Werkzeugangriffsöffnung
- 19: Öffnung (Ausleger)

## Patentansprüche

1. Implantat (IM) zur flächigen Verbindung von Gewebe mit Knochen, wobei das Implantat (IM) eine Klemmfläche (1) umfasst,
wobei die Klemmfläche (1) einen äußeren Rand (3) und einen inneren Rand (4) umfasst,
wobei der äußere Rand (3) zumindest teilweise mittels wenigstens eines Verbindungssteges (5) zumindest teilweise mit dem inneren Rand (4) verbunden ist
und/oder
dass die Klemmfläche (1) einen äußeren Rand (3) und wenigstens eine innerhalb der Klemmfläche (1) angeordnete Öffnung (14) umfasst,
wobei die Öffnung (14) einen inneren Rand (4) umfasst, der mittels wenigstens eines Verbindungssteges (5) zumindest mit einem Teilbereich des äußeren Randes (3) der Klemmfläche (1) verbunden ist
wobei
die Klemmfläche (1) einen kreisrunden oder ovalen oder vieleckigen Umfang, insbesondere eine rotationssymmetrische und/oder spiegelsymmetrische Kontur aufweist,
wobei die Klemmfläche (1) eine weitere Öffnung (7', 7") aufweist, wobei die weitere Öffnung (7', 7") zur Aufnahme eines Befestigungsmittels (2') ausgebildet ist,
wobei das Befestigungsmittel (2') einen Stift, einen Kopf und einen Hals umfasst,
wobei die Klemmfläche (1) wenigstens einen Ausleger (15) umfasst, wobei der Ausleger (15) zu der Klemmfläche (1) gekröpft ausgebildet ist, wobei der Ausleger (15) wenigstens eine Öffnung (16, 19) aufweist, wobei die Öffnung des Auslegers (19) zur Aufnahme eines Befestigungsmittels (2") ausgebildet ist und wobei eine weitere Öffnung des Auslegers (16) als Aussparung ausgebildet ist,
wobei die Klemmfläche (1) mehrere Fixiermittel (6', 6", 6‴, 6ʺʺ) umfasst, wobei die Fixiermittel (6', 6", 6 " `, 6ʺʺ) an einer Unterseite der Klemmfläche (1), insbesondere senkrecht zu der Klemmfläche (1), ausgebildet sind
**dadurch gekennzeichnet, dass** die Fixiermittel (6', 6", 6‴, 6ʺʺ) mit einer Länge von mehr als 2 Millimeter, bevorzugt mit einer Länge zwischen 2 und 6 Millimeter derart ausgebildet sind, dass durch ihre Durchdringung des Gewebes und der danach folgenden Abstützung am Knochen ein zu hoher Anpressdruck des Implantats verhindert und gleichzeitig eine besonders sichere bzw. stabile Fixierung von Gewebe auf Knochen gewährleistet wird.

2. Implantat (IM) nach Anspruch 1 **dadurch gekennzeichnet, dass** die Länge der Fixiermittel (6', 6", 6‴, 6ʺʺ) zum äußeren Rand der Klemmfläche (1) zunimmt.

3. Implantat (IM) nach wenigstens einem der vorhergehenden Ansprüche **dadurch gekennzeichnet, dass** die weitere Öffnung (7', 7") eine Senke zur formschlüssigen Aufnahme des Kopfes (8) aufweist.

4. Implantat (IM) nach wenigstens einem der vorhergehenden Ansprüche **dadurch gekennzeichnet, dass** der Hals (9) und der Kopf (8) mit der weiteren Öffnung (7', 7") ein Gelenk, insbesondere ein taumelgelenkartiges Gelenk, ausbilden.

5. Implantat (IM) nach wenigstens einem der vorhergehenden Ansprüche **dadurch gekennzeichnet, dass** das Gelenk ein Verkippen der Klemmfläche (1) zu einer durch die Längsachse des Stiftes (12) definierten Achse um zwischen 0° und 65°, insbesondere 0° und 55°, bevorzugt 0° und 45°, besonders bevorzugt zwischen 0° und 20° ermöglicht.

6. Implantat (IM) nach wenigstens einem der vorhergehenden Ansprüche **dadurch gekennzeichnet, dass** die weitere Öffnung (7") in der Klemmfläche (1) exzentrisch ist.

7. Implantat (IM) nach wenigstens einem der vorhergehenden Ansprüche **dadurch gekennzeichnet, dass** die Klemmfläche (1) wenigstens zwei sich gegenüberliegende Dorne (17', 17") umfasst, wobei die Dorne (17', 17") an der Unterseite der Klemmfläche (1) ausgebildet sind und/oder wobei die Dorne (17', 17") bevorzugt Widerhaken umfassen.

8. Implantat (IM) nach wenigstens einem der vorhergehenden Ansprüche **dadurch gekennzeichnet, dass** der Stift (10) ein Gewinde umfasst.

9. Implantat (IM) nach wenigstens einem der vorhergehenden Ansprüche **dadurch gekennzeichnet, dass** der Stift (10) hülsenförmig, insbesondere nach der Art einer Einschlagshülse ausgebildet ist und/oder dass der Stift (10) Widerhaken umfasst.

10. Implantat (IM) nach wenigstens einem der vorhergehenden Ansprüche **dadurch gekennzeichnet, dass** die Fixiermittel (6', 6", 6‴, 6ʺʺ) material-einstückig an der Klemmfläche (1) ausgebildet sind und/oder dass die Fixiermittel (6', 6", 6‴, 6ʺʺ) kegelförmig ausgebildet sind und/oder dass die Fixiermittel (6', 6", 6‴, 6ʺʺ) flächendicht ausgebildet sind.

11. Implantat (IM) nach wenigstens einem der vorhergehenden Ansprüche, **dadurch gekennzeichnet, dass** wenigstens zwei Befestigungsmittel (2', 2") in etwa parallel zueinander angeordnet sind und/oder unterschiedliche Längen aufweisen und/oder dass die Klemmfläche (1) eine Ebene bildet, wobei wenigstens ein Befestigungsmittel (2', 2") in etwa senkrecht zu der Ebene und/oder wenigstens ein Befestigungsmittel (2', 2") in etwa parallel zu der Ebene angeordnet ist.

12. Implantat (IM) nach wenigstens einem der vorhergehenden Ansprüche, **dadurch gekennzeichnet, dass** die Klemmfläche (1) eine Werkzeugangriffsfläche und/oder Werkzeugangriffsöffnung (18) umfasst, wobei die Werkzeugangriffsfläche frei von Fixiermitteln ist.

13. Implantat (IM) nach wenigstens einem der vorhergehenden Ansprüche, **dadurch gekennzeichnet, dass** der wenigstens eine Ausleger (15) etwa entlang einer Quererstreckung der Klemmfläche (1) angeordnet ist und/oder dass der wenigstens eine Ausleger (15) und die Klemmfläche (1) einstückig ausgestaltet sind.

14. Implantat (IM) nach wenigstens einem der vorhergehenden Ansprüche, **dadurch gekennzeichnet, dass** die Klemmfläche (1) einen zumindest abschnittsweise gerundeten Umfang aufweist und/oder zumindest abschnittsweise gewölbt ist.

## Claims

1. Implant (IM) for planar connection of tissue to bone, wherein the implant (IM) comprises a clamping surface (1),
wherein the clamping surface (1) comprises an outer edge (3) and an inner edge (4),
wherein the outer edge (3) is connected at least partially to the inner edge (4) at least partially by means of at least one connecting web (5),
and/or
in that the clamping surface (1) comprises an outer edge (3) and at least one opening (14) arranged within the clamping surface (1),
wherein the opening (14) comprises an inner edge (4) which is connected by means of at least one connecting web (5) at least to a partial area of the outer edge (3) of the clamping surface (1),
wherein
the clamping surface (1) has a circular or oval or polygonal circumference, in particular a rotationally symmetrical and/or mirror-symmetrical contour,
wherein the clamping surface (1) has a further opening (7', 7"), wherein the further opening (7', 7'') is designed for receiving a securing means (2'), wherein the securing means (2') comprises a pin, a head and a neck,
wherein the clamping surface (1) comprises at least one projection (15), wherein the projection (15) is offset with respect to the clamping surface (1), wherein the projection (15) has at least one opening (16, 19), wherein the opening in the projection (19) is designed for receiving a securing means (2''), and wherein a further opening in the projection (16) is designed as a cutout,
wherein the clamping surface (1) comprises a plurality of fixing means (6', 6", 6‴, 6ʺʺ), wherein the fixing means (6', 6", 6‴, 6ʺʺ) are formed on an underside of the clamping surface (1), in particular perpendicularly to the clamping surface (1) **characterized in that** the fixing means (6', 6", 6‴, 6ʺʺ) are formed with a length of more than 2 millimeters, preferably with a length of between 2 and 6 millimeters in such a manner that, by the fixing means penetrating the tissue and subsequently being supported on the bone, an excessive contact pressure of the implant is prevented and at the same time a particularly secure and stable fixing of tissue on bone is ensured.

2. Implant (IM) according to Claim 1, **characterized in that** the length of the fixing means (6', 6", 6‴, 6ʺʺ) increases towards the outer edge of the clamping surface (1) .

3. Implant (IM) according to at least one of the preceding claims, **characterized in that** the further opening (7', 7") has a hollow for receiving the head (8) with a form fit.

4. Implant (IM) according to at least one of the preceding claims, **characterized in that** the neck (9) and the head (8) with the further opening (7', 7'') form a joint, in particular a joint in the manner of a wobble joint.

5. Implant (IM) according to at least one of the preceding claims, **characterized in that** the joint permits tilting of the clamping surface (1) with respect to an axis, which is defined by the longitudinal axis of the pin (12), by between 0° and 65°, in particular 0° and 55°, preferably 0° and 45°, particularly preferably between 0° and 20°.

6. Implant (IM) according to at least one of the preceding claims, **characterized in that** the further opening (7'') in the clamping surface (1) is eccentric.

7. Implant (IM) according to at least one of the preceding claims, **characterized in that** the clamping surface (1) comprises at least two opposite spikes (17', 17''), wherein the spikes (17', 17'') are formed on the underside of the clamping surface (1), and/or wherein the spikes (17', 17'') preferably comprise barbs.

8. Implant (IM) according to at least one of the preceding claims, **characterized in that** the pin (10) comprises a thread.

9. Implant (IM) according to at least one of the preceding claims, **characterized in that** the pin (10) is configured in the form of a sleeve, in particular in the manner of a drive-in sleeve, and/or **in that** the pin (10) comprises barbs.

10. Implant (IM) according to at least one of the preceding claims, **characterized in that** the fixing means (6', 6", 6‴, 6ʺʺ) are integrally formed on the clamping surface (1) in the same material, and/or **in that** the fixing means (6', 6", 6‴, 6ʺʺ) are cone-shaped, and/or **in that** the fixing means (6', 6", 6‴, 6ʺʺ) are surface-tight.

11. Implant (IM) according to at least one of the preceding claims, **characterized in that** at least two securing means (2', 2") are arranged approximately parallel to one another and/or have different lengths, and/or **in that** the clamping surface (1) forms a plane, wherein at least one securing means (2', 2") is arranged approximately perpendicular to the plane and/or at least one securing means (2', 2'') is arranged approximately parallel to the plane.

12. Implant (IM) according to at least one of the preceding claims, **characterized in that** the clamping surface (1) comprises a tool engagement surface and/or tool engagement opening (18), the tool engagement surface being free from fixing means.

13. Implant (IM) according to at least one of the preceding claims, **characterized in that** the at least one projection (15) is arranged approximately along a transverse extent of the clamping surface (1), and/or **in that** the at least one projection (15) and the clamping surface (1) are formed in one piece.

14. Implant (IM) according to at least one of the preceding claims, **characterized in that** the clamping surface (1) has an at least partially rounded circumference and/or is at least partially curved.

## Revendications

1. Implant (IM) pour une liaison à plat d'un tissu avec un os, l'implant (IM) comprenant une surface de serrage (1),
la surface de serrage (1) comprenant un bord extérieur (3) et un bord intérieur (4),
le bord extérieur (3) étant au moins partiellement relié au moins partiellement au bord intérieur (4) au moyen d'au moins une bande de liaison (5)
et/ou
la surface de serrage (1) comprenant un bord extérieur (3) et au moins une ouverture (14) aménagée à l'intérieur de la surface de serrage (1),
l'ouverture (14) comprenant un bord intérieur (4) qui est relié à au moins une partie du bord extérieur (3) de la surface de serrage (1) au moyen d'au moins une bande de liaison (5),
dans lequel
la surface de serrage (1) présente un périmètre circulaire ou ovale ou polygonale, en particulier un contour à symétrie de révolution et/ou à symétrie miroir,
la surface de serrage (1) présentant une autre ouverture (7', 7"), l'autre ouverture (7', 7") étant conçue pour recevoir un moyen de fixation (2'), le moyen de fixation (2') comprenant une tige, une tête et un col,
la surface de serrage (1) comprenant au moins un bras (15), le bras (15) étant réalisé de façon coudé par rapport à la surface de serrage (1), le bras (15) présentant au moins une ouverture (16, 19), l'ouverture (19) du bras étant conçue de façon à recevoir un moyen de fixation (2") et une autre ouverture (16) du bras étant réalisée sous forme d'évidement,
la surface de serrage (1) comprenant une pluralité de moyens de fixation (6', 6", 6‴, 6ʺʺ), les moyens de fixation (6', 6", 6‴, 6ʺʺ) étant formés sur une face inférieure de la surface de serrage (1), en particulier perpendiculairement à la surface de serrage (1),
**caractérisé en ce que** les moyens de fixation (6', 6", 6‴, 6ʺʺ), d'une longueur supérieure à 2 millimètres, de préférence d'une longueur comprise entre 2 et 6 millimètres, sont conçus de telle sorte que leur pénétration dans le tissu et leur appui consécutif sur l'os empêchent une pression d'application trop élevée de l'implant et garantissent en même temps une fixation particulièrement sûre ou stable du tissu sur l'os.

2. Implant (IM) selon la revendication 1, **caractérisé en ce que** la longueur des moyens de fixation (6', 6", 6‴, 6ʺʺ) augmente en direction du bord extérieur de la surface de serrage (1).

3. Implant (IM) selon au moins une des revendications précédentes, **caractérisé en ce que** l'autre ouverture (7', 7") présente un creux pour la réception de la tête (8) par complémentarité de forme.

4. Implant (IM) selon au moins une des revendications précédentes, **caractérisé en ce que** le col (9) et la tête (8) forment, avec ladite autre ouverture (7', 7"), une articulation, en particulier une articulation oscillante.

5. Implant (IM) selon au moins une des revendications précédentes, **caractérisé en ce que** l'articulation permet d'incliner la surface de serrage (1) par rapport à un axe défini par l'axe longitudinal de la tige (12), d'entre 0° et 65°, en particulier d'entre 0° et 55°, de préférence d'entre 0° et 45°, de manière particulièrement préférée d'entre 0° et 20°.

6. Implant (IM) selon au moins une des revendications précédentes, **caractérisé en ce que** ladite autre ouverture (7") dans la surface de serrage (1) est excentrée.

7. Implant (IM) selon au moins une des revendications précédentes, **caractérisé en ce que** la surface de serrage (1) comprend au moins deux mandrins (17', 17") opposés, les mandrins (17', 17") étant formés sur la face inférieure de la surface de serrage (1) et/ou les mandrins (17', 17") comprenant de préférence des barbillons.

8. Implant (IM) selon au moins une des revendications précédentes, **caractérisé en ce que** la tige (10) comprend un filetage.

9. Implant (IM) selon au moins une des revendications précédentes, **caractérisé en ce que** la tige (10) est réalisée en forme de douille, en particulier à la manière d'une douille à enfoncer et/ou **en ce que** la tige (10) comprend des picots.

10. Implant (IM) selon au moins une des revendications précédentes, **caractérisé en ce que** les moyens de fixation (6', 6", 6‴, 6ʺʺ) sont réalisés en une seule pièce de matériau sur la surface de serrage (1) et/ou **en ce que** les moyens de fixation (6', 6", 6‴, 6ʺʺ) sont réalisés en forme de cône et/ou **en ce que** les moyens de fixation (6', 6", 6‴, 6ʺʺ) sont réalisés de manière imperméable en surface.

11. Implant (IM) selon au moins une des revendications précédentes, **caractérisé en ce qu'**au moins deux moyens de fixation (2', 2") sont disposés à peu près parallèlement l'un à l'autre et/ou présentent des longueurs différentes et/ou **en ce que** la surface de serrage (1) forme un plan, au moins un moyen de fixation (2', 2") étant agencé à peu près perpendiculairement au plan et/ou au moins un moyen de fixation (2', 2") étant agencé à peu près parallèlement au plan.

12. Implant (IM) selon au moins une des revendications précédentes, **caractérisé en ce que** la surface de serrage (1) comprend une surface d'engagement d'outil et/ou une ouverture (18) d'engagement d'outil, la surface d'engagement d'outil étant exempte de moyens de fixation.

13. Implant (IM) selon au moins une des revendications précédentes, **caractérisé en ce que** ledit au moins un bras (15) est disposé approximativement le long d'une extension transversale de la surface de serrage (1) et/ou **en ce que** ledit au moins un bras (15) et la surface de serrage (1) sont réalisés d'une seule pièce.

14. Implant (IM) selon au moins une des revendications précédentes, **caractérisé en ce que** la surface de serrage (1) présente une périphérie au moins partiellement arrondie et/ou est au moins partiellement bombée.
